## Europäisches Patentamt
## European Patent Office
## Office européen des brevets

(11) Publication number: **0 147 133**
**B1**

(12) **EUROPEAN PATENT SPECIFICATION**

(45) Date of publication of patent specification: **08.02.89**

(21) Application number: **84308670.3**

(22) Date of filing: **13.12.84**

(51) Int. Cl.⁴: **C 07 C 31/20, C 07 C 29/00**

(54) **Process for the production of alkylene glycols from secondary alcohols.**

(30) Priority: **30.12.83 GB 8334612**

(43) Date of publication of application:
**03.07.85 Bulletin 85/27**

(45) Publication of the grant of the patent:
**08.02.89 Bulletin 89/06**

(84) Designated Contracting States:
**AT BE DE FR GB IT LU NL SE**

(56) References cited:
**US-A-4 412 084**

**PATENT ABSTRACTS OF JAPAN, vol. 8, no. 225
(C-247)1662r, 16th October 1984; & JP - A - 59
110 638 (NIPPON SHOKUBAI KAGAKU KOGYO)
26-06-1984**

**PATENT ABSTRACTS OF JAPAN, vol. 7, no. 259
(C-195)1404r, 18th November 1983; & JP - A -
58 144 331 (KOGYO GIJUTSUIN) 27-08-1983**

(73) Proprietor: **The British Petroleum Company
p.l.c.
Britannic House Moor Lane
London EC2Y 9BU (GB)**

(72) Inventor: **Griggs, Colin George The B.P.C.p.l.c.
Chertsey Road Sunbury-on-Thames
Middlesex TW16 7LN (GB)**
Inventor: **Pippard, David Alan The B.P.C. p.l.c.
Chertsey Road
Sunbury-on-Thames Middlesex TW 16 7LN (GB)**

(74) Representative: **MacLeod, Malcolm et al
BP INTERNATIONAL LIMITED Patents Division
Chertsey Road
Sunbury-on-Thames Middlesex TW16 7LN (GB)**

Courier Press, Leamington Spa, England.

## Description

The present invention relates to a process for the production of alkylene glycols having 3 or more carbon atoms from secondary alcohols.

Alkylene glycols in general are important industrial chemicals. Thus, the simplest glycol, ethylene glycol is a very large tonnage industrial chemical which finds direct use in automotive coolant and antifreeze solutions, as a heat-transfer agent in refrigeration, and as an ingredient of deicing fluids for airport runways. Its uses as a chemical intermediate include its incorporation as an essential constituent in polyester fibres, films and bottle resins. It is also used as a solvent in for example lacquers, printing inks and adhesives.

In recent years, emphasis has shifted away from traditional methods of manufacturing ethylene glycol towards routes which utilise synthesis gas (gaseous mixtures comprising carbon monoxide and hydrogen), largely because synthesis gas can be derived not only from petroleum but also from such raw materials as natural gas and coal, and potentially from oil shale and tar sands. Thus, according to US Patents Nos. 2,316,564; 2,153,064; 2,152,852; 2,285,448; and 2,331,094, ethylene glycol can be produced by reacting formaldehyde with carbon monoxide and water at high pressures (over 300 bars) in the presence of an acid catalyst to produce hydroxyacetic (glycollic) acid, reacting the acid so-produced with methanol to form the methyl ester and thereafter converting the methyl ester to ethylene glycol by catalytic hydrogenation. Another process, disclosed in US Patents Nos. 4,115,428 and 4,115,433, describes the production of ethylene glycol by reacting methanol and carbon monoxide at high pressures using a rhodium catalyst.

Even more recently, GB patent applications publication Nos. 2083037 and 2083038, US Patent No. 4,393,252 and European patent publication Nos. 71457 and 71458 relating to the production of ethylene glycol by reacting methanol, formaldehyde and an oorganic peroxide have appeared. GB—A—2083038 discloses a process for producing ethylene glycol by reacting methanol, an organic peroxide, and formaldehyde in the presence of water (suitably from about 0.5 to about 35 weight per cent), said organic peroxide having the formula R—O—O—R$^1$ wherein R and R$^1$ each is an alkyl or aralkyl group containing 3 to 12 carbon atoms, wherein no more than about 6 per cent of organic peroxide, based on the total weight of methanol, organic peroxide, formaldehyde and water present, is utilised in the initial reaction mixture. GB—A—2083037 described a similar process in which a basic material is added to the reactants in an amount sufficient to reduce the hydrogen ions that are being formed in the reaction without unduly reducing the ethylene glycol production due to by-product formation. In Examples 13 and 14 of GB—A—2083037 the monomeric aqueous formaldehyde was produced from paraformaldehyde by adding a small amount of hydrogen chloride to depolymerise the paraformaldehyde to aqueous monomeric formaldehyde and thereafter the formaldehyde solution was neutralized by the addition of a small amount of sodium bicarbonate. USP 4,393,252 discloses subject matter similar to that of GB—A—2083037. EP—A—71457 discloses the improvement wherein the formaldehyde and the organic peroxide are added portionwise at intervals throughout the reaction period. Finally EP—A—71458 discloses the improvement wherein the amount of the peroxide used is greater than 6 and up to 25 weight percent and the amounts of water used is from 0.5 to 36 weight percent, and the amount of the peroxide and water used are dependent on each other so that either the amount of the peroxide used is greater than 6 up to 12 weight percent and the amount of water used is from 0.5 to 35 weight percent, or the amount of the peroxide used is greater than 12 up to 15 weight percent and the amount of water used is from 0.5 to 25 weight percent, or the amount of the peroxide used is greater than 15 up to 20 weight percent and the amount of water used is from 0.5 to 15 weight percent, or the amount of the peroxide used is greater than 20 up to 25 weight percent and the amount of water used is from 0.5 to 10 weight percent, the above weight percentages being based on the total weight of the methanol, organic peroxide, formaldehyde and water present in the reaction mixture.

Our copending European patent application No. 110666 describes a process for the production of ethylene glycol which process comprises reacting at elevated temperature methanol, a polymeric source of formaldehyde and an organic peroxide having the formula R—O—O—R$^1$ wherein R and R$^1$ are independently either alkyl or aralkyl groups containing from 3 to 12 carbon atoms.

Although most of the research carried out to date has had the aim of producing ethylene glycol from synthesis gas derived feedstocks, it is becoming increasingly important to develop alternative, cheap routes to higher alkylene glycols having three or more carbon atoms. A problem however arises when methanol is replaced by another primary alcohol in a process of the type described in GB—A—2083037, since under the conditions of reaction the primary alcohol is rapidly oxidised to an aldehyde by the organic peroxide. As this oxidation is faster than the reaction between the primary alcohol and the formaldehyde, only trace amounts of the higher molecular weight polyfunctional alcohol are produced. Thus, for example, if the higher alcohol used is ethanol, the main reaction product is acetaldehyde with little or no propane-1,2-diol being produced.

Surprisingly, it has now been found that when the primary alcohol is replaced by a secondary alcohol, direct oxidation of the alcohol is reduced and the organic peroxide effects the reaction between the secondary alcohol and the formaldehyde to produce a higher alkylene glycol having three or more carbon atoms in high yields. In addition the higher alkylene glycol there is also produced the alcohol derived from the organic peroxide by cleavage.

Accordingly, the present invention provides a process for the production of a higher alkylene glycol having three or more carbon atoms from a secondary alcohol which process comprises reacting at elevated temperature the secondary alcohol with formaldehyde, and an organic peroxide having the formula R—O—O—R$^1$ wherein R and R$^1$ are independently either alkyl or aralkyl groups containing from 3 to 12 carbon atoms.

It is an important feature of the invention that a secondary alcohol is used as a feedstock, since primary alcohols, with the exception of methanol, are rapidly oxidised under the reaction conditions and tertiary alcohols are substantially unreactive. Any secondary alcohol may be used as long as it has at least one secondary hydroxyl group and no primary hydroxyl groups. Thus, a secondary alcohol having two or more secondary hydroxyl groups or a secondary alcohol having one secondary hydroxyl group and one tertiary hydroxyl group are both examples of possible classes of feedstock. The secondary alcohol, however, is preferably a monofunctional secondary alcohol having between 3 and 20 carbon atoms. Preferred monofunctional secondary alcohols include propan-2-ol, butanol-2-ol, pentan-2-ol, pentan-3-ol, hexan-2-ol, hexan-3-ol, hexan-4-ol, 3-methyl, pentan-2-ol, cyclohexanol, cycloheptanol and the like. In the case of propan-2-ol the reaction product is 2-methyl, propane-1,2-diol.

Formaldehyde may be added as an aqueous solution, for example as formation, a polymeric solid or as gaseous formaldehyde generated by thermal depolymerisation of a formaldehyde polymer.

Suitable polymeric sources of formaldehyde include paraformaldehyde, otherwise known as paraform, trioxane and tetraoxane, of which paraformaldehyde is preferred. Paraformaldehyde is a solid mixture of linear poly(oxymethylene glycols) of relatively short chain length and may be represented by the formula HO(CH$_2$O)$_n$H, wherein n is 8—100. Commercially available forms of paraformaldehyde generally have an average molecular weight of about 600 and may contain up to about 9 wt% water and a maximum acidity as formic acid of 0.03 wt%. Such commercially available forms may be used in the process of the present invention without further purification. Alternatively, the commercially available forms may be further purified before use in the process. If desired, higher molecular weight, i.e. n greater than 100, suitably in the range from 100 to 500, forms of polyoxymethylene glycols of the formula HO(CH$_2$O)$_n$H may be employed. Trioxane, which may also be used in the process of the present invention is the cyclic symmetrical trimer of formaldehyde and is also a solid. Trioxane is also commercially available. Tetraoxane, which is a cyclic tetramer of formaldehyde, is a solid.

The organic peroxide having the formula R—O—O—R$^1$ wherein R and R$^1$ are independently either alkyl or aralkyl groups containing from 3 to 12 carbon atoms may suitably be di-tertiary-butyl peroxide, di-cumyl peroxide, tertiary-butyl cumyl peroxide or tertiary-butyl ethyl benzylperoxide. Preferably the peroxide is either di-cumyl peroxide or ditertiary-butyl peroxide.

The process may be operated in the presence of water, using reactants which have not been dried, optionally with additional water. Thus a typical feed may comprise from about 45 to about 97% by weight, preferably from about 80 to about 90% by weight of secondary alcohol, from about 0.5 to about 50% by weight, preferably from about 2 to about 12% by weight of formaldehyde or the polymeric source of formaldehyde, from about 0.25 to about 15% by weight, preferably from about 0.75 to about 3% by weight of organic peroxide and from about 0.5 to about 35% by weight of water, all percentages by weight being based on the total weight of the reactants.

However, as mentioned hereinabove, it is preferred to operate the process of the invention under substantially anhydrous conditions, i.e. at a water content of less than 0.5% by weight, preferably less than 0.25% by weight, based on the total weight of the reactants. It will be found convenient when operating under substantially anhydrous conditions to employ a polymeric source of formaldehyde. It may be necessary when operating under substantially anhydrous conditions to use at least partially dried reactants.

As regards the reaction conditions, the process may suitably be operated at a temperature greater than 100°C, for example in the range 100 to 200°C, preferably from 125 to 175°C. Under certain conditions, it may be desirable to operate at a temperature below 100°C. The process may be operated at autogenous pressure, that is the pressure generated in a closed reactor at the particular reaction temperature, though pressures above and below autogenous may be used if so desired. Generally, the reaction time for a batch operation may suitably be in the range from 0.25 to 8 hours, preferably from 0.5 to 4 hours.

The process may be operated batchwise, semi-continuously or continuously, preferably continuously. Suitable methods of operation are described in the aforesaid EP—A—71457 and EP—A—71458, for example. Such methods include the portionwise addition of the organic peroxide and formaldehyde or polymeric source thereof at intervals throughout the reaction period. The product mixture may be purified using conventional techniques, such as distillation or solvent extraction, to recover higher alkylene glycol of the desired purity. Besides higher alkylene glycol and alcohol resulting from the cleavage of the peroxide, for example tertiary butanol when the organic peroxide is ditertiary butyl peroxide, there may also be formed small amounts by-products, as might be formed by the condensation of alkyl glycols or their subsequent oxidation.

The invention will now be illustrated by reference to the following Examples. The paraformaldehyde, as used in the Examples, was supplied by Fisons. It conforms with the formula $HO(CH_2O)_nH$ wherein n is in the range from 8 to 100 and has an average value of about 60. The water content of the paraformaldehyde is less than or equal to 2%, the formaldehyde content is about 97% and the rest is methanol.

Example 1

Into a 100 ml autoclave was weighed paraformaldehyde $[(CH_2O)_n]$, di-tertiary-butyl peroxide (DTBP) and propan-2-ol; the autoclave was closed and sealed. The autoclave and contents were then heated to 155°C and held at this temperature for 2 hours. At the end of this time the autoclave was cooled, opened and its contents analysed by GC/MS. Analysis of the product revealed the presence of 2-methyl, propane-1,2-diol in apparent high yield. No traces of acetone, the oxidation product of isopropanol, was detected.

Example 2

The method of Example 1 was repeated except that propan-2-ol was replaced by ethanol. After 2 hours reaction and subsequent cooling, the product mixture was analysed by GC/MS. Analysis of the mixture revealed the presence of large quantities of acetaldehyde. No propane-1,2-diol was detected.

Claims

1. A process for the production of a higher alkylene glycol having three or more carbon atoms from a secondary alcohol which process comprises reacting at elevated temperature the secondary alcohol with formaldehyde and an organic peroxide having the formula $R—O—O—R^1$ wherein R and $R^1$ are independently either alkyl or aralkyl groups containing from 3 to 12 carbon atoms.

2. A process as claimed in Claim 1 wherein the reaction is carried out under substantially anhydrous conditions.

3. A process as claimed in Claim 1 or Claim 2 wherein a polymeric source of formaldehyde is used.

4. A process as claimed in any one of the preceding claims wherein the secondary alcohol is a monofunctional secondary alcohol having between 3 and 20 carbon atoms.

5. A process as claimed in Claim 4 wherein the monofunctional secondary alcohol is selected from the group comprising propan-2-ol, butan-2-ol, pentan-2-ol, pentan-3-ol, hexan-2-ol, hexan-3-ol, hexan-4-ol, and 3-methyl, pentan-2-ol, cyclohexanol and cycloheptanol.

6. A process as claimed in any one of the preceding claims characterised in that the organic peroxide is di-tertiary butyl peroxide or dicumyl peroxide.

7. A process as claimed in any one of the preceding claims wherein the temperature of the reaction is in the range 125 to 175°C.

Patentansprüche

1. Verfahren zur Herstellung eines höheren Alkylenglykols mit 3 oder mehr Kohlenstoffatomen aus einem sekundären Alkohol, welches Verfahren ein Umsetzen des sekundären Alkohols bei erhöhter Temperatur mit Formaldehyd und einem organischen Peroxid mit der Formel $R—O—O—R^1$, worin R und $R^1$ unabhängig voneinander entweder Alkyl- oder Aralkylgruppen mit 3 bis 12 Kohlenstoffatomen bedeuten, umfaßt.

2. Verfahren nach Anspruch 1, worin die Umsetzung unter im wesentlichen wasserfreien Bedingungen ausgeführt wird.

3. Verfahren nach Anspruch 1 oder 2, worin eine polymere Formaldehydquelle verwendet wird.

4. Verfahren nach einem der vorstehenden Ansprüche, worin der sekundäre Alkohol ein einwertiger sekundärer Alkohol mit zwischen 3 und 20 Kohlenstoffatomen ist.

5. Verfahren nach Anspruch 4, worin der einwertige sekundäre Alkohol aus der aus Propan-2-ol, Butan-2-ol, Pentan-2-ol, Pentan-3-ol, Hexan-2-ol, Hexan-3-ol, Hexan-4-ol und 3-Methyl-pentan-2-ol, Cyclohexanol und Cycloheptanol bestehenden Gruppe ausgewählt ist.

6. Verfahren nach einem der vorstehenden Ansprüche, dadurch gekennzeichnet, daß das organische Peroxid Di-tert.butylperoxid oder Dicumylperoxid ist.

7. Verfahren nach einem der vorstehenden Ansprüche, worin die Reaktionstemperatur im Bereich von 125 bis 175°C liegt.

Revendications

1. Procédé pour la production d'un alkylène glycol supérieur comportant trois ou plus de trois atomes de carbone, à partir d'un alcool secondaire, ce procédé comprenant la réaction, à température élevée, de l'alcool secondaire avec le formaldéhyde et avec un peroxyde organique répondant à la formule $R—O—O—R^1$, dans laquelle R et $R^1$ représentent chacun, indépendamment, un groupe alkyle ou aralkyle contenant de 3 à 12 atomes de carbone.

2. Procédé tel que revendiqué à la revendication 1, dans lequel on conduit la réaction dans des conditions essentiellement anhydres.

3. Procédé tel que revendiqué à la revendication 1 ou à la revendication 2, dans lequel on utilise une source polymère de formaldéhyde.

4. Procédé tel que revendiqué dans l'une quelconque des revendications précédentes, dans lequel l'alcool secondaire est un alcool secondaire monofonctionnel ayant entre 3 et 20 atomes de carbone.

5. Procédé tel que revendiqué à la revendication 4, dans lequel l'alcool secondaire monofonctionnel est choisi dans l'ensemble comprenant le propane-2-ol, le butane-2-ol, le pentane-2-ol, le pentane-3-ol, l'hexane-2-ol, l'hexane-3-ol, l'hexane-4-ol et le 3-méthyl-pentane-2-ol le cyclohexanol et le cycloheptanol.

6. Procédé tel que revendiqué dans l'une quelconque des revendications précédentes, caractérisé en ce que le peroxyde organique est le peroxyde de di-tertiobutyle ou le peroxyde de di-cumyle.

7. Procédé tel que revendiqué dans l'une quelconque des revendications précédentes, dans lequel la température de la reaction se situe entre 125 et 175°C.